# EUROPEAN PATENT SPECIFICATION

(11) **EP 3 729 048 B1**
(45) Date of publication and mention of the grant of the patent: **30.08.2023**
(21) Application number: 18830253.3
(22) Date of filing: 20.12.2018
(51) Int. Cl.: G01N 1/30, C12Q 1/04, G01N 33/50

(54) **PHENOL-FREE ACID-FAST STAINING COMPOSITION AND USE THEREOF**
PHENOL-FREIE, SÄUREFESTE FÄRBEMITTELZUSAMMENSETZUNG UND DEREN VERWENDUNG
COMPOSITION DE COLORATION RÉSISTANT AUX ACIDES SANS PHÉNOL ET SON UTILISATION

(30) Priority: 24.12.2017 US 201762610215 P
(43) Date of publication of application: 28.10.2020
(73) Proprietor: Ventana Medical Systems, Inc., Tucson, Arizona 85755 (US)
(72) Inventor: MEHTA, Parula, Tucson, Arizona 85755 (US); KLEIN, Eric, Tucson, Arizona 85755 (US); SACCHETTI, Dominic, Tucson, Arizona 85755 (US); KESHWANI, Malik, Tucson, Arizona 85755 (US); KOSMEDER II, Jerome W., Tucson, Arizona 85755 (US)
(74) Representative: Teschemacher, Andrea
(86) International application number: PCT/EP2018/086069
(87) International publication number: WO 2019/122062

(56) References cited:
- EP-A2- 0 286 255
- WO-A1-95/17519
- WO-A1-2016/170008
- US-A1- 2004 089 847
- US-B2- 7 410 753
- R C ELLIS ET AL: "Safer staining method for acid fast bacilli.", JOURNAL OF CLINICAL PATHOLOGY, vol. 46, no. 6, 1 June 1993 (1993-06-01), pages 559-560, XP055556076, GB ISSN: 0021-9746, DOI: 10.1136/jcp.46.6.559
- Newcomersupply: "AFB, Basic Fuchsin, Ellis & Zabrowarny Stain Kit -Technical Memo", , 30 November 2017 (2017-11-30), XP055556103, Retrieved from the Internet: URL:http://www.newcomersupply.com/document s/staining/staining-kits/Kit%20AFB%20Basic %20Fuchsin.91012.pdf [retrieved on 2019-02-13]
- CLARKE S C ET AL: "MODIFIED DETERGENT ZIEHL-NEELSEN TECHNIQUE FOR THE STAINING OF CYCLOSPORA CAYETANENSIS", JOURNAL OF CLINICAL PATHO, BMJ PUBLISHING GROUP, GB, vol. 49, no. 6, 1 January 1996 (1996-01-01), page 511/512, XP009044404, ISSN: 0021-9746, DOI: 10.1136/JCP.49.6.511
- D.K. Raoul, O.S. Johnbull & I. John: "A faster and safer staining technique for acid fas bacilli in resouce-poor setting", Int. J. Medicine and Medical Sciences , vol. 1, no. 5 31 May 2009 (2009-05-31), pages 238-240, XP002788870, Retrieved from the Internet: URL:https://www.researchgate.net/publicati on/237605163_A_faster_and_safer_staining_t echnique_for_acid_fast_bacilli_in_resource -poor_setting [retrieved on 2019-02-13]
- Anonymous: "Multi-Purpose Cleaner L.O.C. (TM) | Amway", , 31 December 2011 (2011-12-31), pages 1-2, XP055826356, Retrieved from the Internet: URL:https://www.amway.ro/en/product/0001,% 20multi-purpose-cleaner-l-o-c?code=en<>pro duct_ingredients.#product_ingredients [retrieved on 2021-07-21]

## Description

### BACKGROUND OF THE DISCLOSURE

The lipid containing cell walls of mycobacteria, such as M. tuberculosis, have the unique characteristic of binding carbolfuchsin stain so tightly that they resist destaining with strong decolorizing agents such as alcohol and strong acids. Thus, the term "acid-fast" has been used to describe a carbolfuchsin staining reaction for certain types of bacilli, such as mycobacteria which resists destaining by acids or alcohol. The acid-fast staining reaction of mycobacteria, along with their unique beaded and slightly curved shape, is a valuable aid in the early detection of infection and monitoring of therapy. The finding of acid-fast bacilli in the sputa or other mycobacteriological specimens is considered presumptive evidence of active tuberculosis and is sufficient to initiate therapy.

In suspected cases of tuberculosis, acid-fast staining techniques are used, since mycobacteria and other acid-fast organisms cannot be stained by gram stain. Slides containing biological samples (e.g. smears) are then treated by the Ziehl-Neelsen method. In the Ziehl-Neelsen method a carbolfuchsin stain is first prepared. The carbolfuchsin stain contains 0.3 grams of basic fuchsin, 10.0 milliliters of ethyl alcohol, and 90 milliliters of 5% aqueous solution of phenol. The carbolfuchsin stain is applied to the slide smears for 5 minutes, applying enough heat for gentle steaming. The stain is not permitted to evaporate, and more stain is added as needed. The slides are then cooled and rinsed in water. The slides are then decolorized in a solution of 95% ethyl alcohol that contains 3% by volume of concentrated hydrochloric acid. A decolorizing solution is added until no more carbolfuchsin stain comes off. The slides are washed in tap water and are then counter-stained with a methylene blue solution for 1-2 minutes. The methylene blue solution contains 0.3 grams of methylene blue (90% dye content) and 100 ml of distilled water. The slides are then washed, dried and examined by a pathologist.

Traditional formulations used to stain acid fast organisms, such as those used in the Ziel-Neelsen method, include fuchsin and phenol. The use of phenol presents several health hazards. For instance, phenol is toxic, generally causing protein-degenerating effects upon exposure. Symptoms of exposure to small amounts can range from skin burns and lung edema to dysrhythmia, seizures, and coma. Long-term exposure is known to cause liver and kidney damage. Besides its toxicity, phenol is caustic and suspected of being carcinogenic. Considerable safety precautions therefore have to be taken during the preparation and use of phenol-containing staining solutions. In addition, phenol has an unpleasant odor, which is usually still present in the end product and easily adheres to hands and clothes.

Ellis and Zabrowarny (J Clin Pathol. 1993; 46(6):559-560) disclose a method for staining acid fast bacilli with a staining solution preheated to 60°C, the staining solution previously mixed together from fuchsin in ethyl alcohol, water and a commercially available mixture of surfactants (see https://www.amway.ro/en/product/0001, multi-purpose-cleaner-l-o-c?code=enproduct_ingredients).

A similar aqueous staining solution made of carbol fuchsin, alocohol and a commercially available detergent is described in Clarke and McIntyre (J Clin Pathol. 1996; 49(6):511-512) for assessing the quality of staining of the parasite Cyclospora cayetanensis in a modified Ziehl-Neelsen technique.

Raoul et al. (Int. J. Med. Med. Sci. 2009; 1(5):238-240) are also concerned with a modified Ziehl-Neelsen method using fuchsin, ethyl alcohol, water and "Morning fresh", a commercial liquid dish washing solution consisting of water, anionic surfactants, hydrotropes, magnesium salt, perfume, preservatives and color.

US 2004/0089847 discloses another phenol-free staining composition comprising fuchsin, ethanol and an organic compound as phenol-substitute.

WO 95/17519 discloses staining compositions for the selective detection of gram +/- bacteria. A solution comprising fuchsin, ethanol and a buffering agent is used as counterstain. The composition is not phenol-free.

WO 2016/170008 and US 7,410,753 disclose an automated dispensing apparatus for providing reagents for an (immuno)histochemical analysis of biological samples on microscopy slides. They also disclose primary staining compositions comprising a dye (hematoxylin or eosin) and a non-ionic surfactant.

### BRIEF SUMMARY OF THE DISCLOSURE

Applicants have developed a phenol-free acid fast staining composition that achieves a staining performance at least equal to those traditional formulations which include phenol (e.g. Ziehl-Neelsen stains). Indeed, the phenol-free compositions described herein achieve at least the same frequency of staining (i.e. the total number of microorganisms stained within a defined area of tissue) and/or stain intensity (i.e. the darkness of stain) as compared with phenol-containing formulations, without the concomitant risk of phenol exposure.

Moreover, Applicants have discovered that the removal of phenol from the staining composition and its replacement with a base and a surfactant permits increased solubility of the fuchsin dye in the staining solution. Increased fuchsin solubility allows for lower concentrations of fuchsin to be used in any staining solution, and this is particularly important for those staining solutions utilized in automated staining instruments where high concentrations of fuchsin could potentially precipitate in the instrument. Finally, removal of phenol, which has a higher vapor pressure, is believed to reduce or eliminate the risk of cross-contamination in automated staining instruments.

In view of the foregoing, in one aspect of the present invention is an acid fast staining composition comprising a fuchsin, a weak base having a pKa ranging from between about 8 to about 20, a non-ionic surfactant, and an alcohol, wherein an amount of fuchsin (e.g. new fuchsin) in the composition ranges from between about 0.75 w/v% to about 2.75 w/v% by total volume of the composition, wherein an amount of base in the composition ranges from between about 0.05 w/v% to about 0.5 w/v% by total volume of the composition, wherein , an amount of surfactant in the composition ranges from between about 0.5 w/v% to about 4 w/v% by total volume of the composition, and wherein the acid fast staining compositions is free from phenol.

In some embodiments, the base is a hydroxide. In some embodiments, the base is a non-nucleophilic base. In some embodiments, the base is tris(hydroxymethyl)aminomethane.

In some embodiments, the non-ionic surfactant is an alcohol ethoxylate. In some embodiments, the non-ionic surfactant is a C₈-C₁₈ alcohol ethoxylate. In some embodiments, the C₈-C₁₈ alcohol ethoxylate comprises less than 12 moles of ethylene oxide.

In some embodiments, a 10% aqueous solution of the acid fast staining composition has a pH ranging from between about 2 to about 6. In some embodiments, the acid fast staining composition is stable for at least 120 days. In some embodiments, the acid fast staining composition is stable for at least 240 days. In some embodiments, the acid fast staining composition is stable for at least 360 days. In some embodiments, the acid fast staining composition is stable for at least 420 days. In some embodiments, the acid fast staining composition is stable for at least 500 days. In some embodiments, the acid fast staining composition is stable for at least 540 days. In some embodiments, the acid fast staining composition is stable for at least 600 days. In some embodiments, the acid fast staining composition is stable for at least 620 days. In some embodiments, the acid fast staining composition further comprises at least one additive.

In some embodiments, the acid fast staining composition consists essentially of (a) new fuchsin in an amount ranging from between about 0.75 w/v% to about 2.75w/v% by total volume of the composition; (b) a weak base in an amount ranging from between about 0.05 w/v% to about 0.5 w/v% by total volume of the composition; and (c) a non-ionic surfactant in an amount ranging from between about 0.5w/v% to about 4w/v% by total volume of the composition. In some embodiments, the surfactant is a C₈-C₁₈ alcohol ethoxylate, and the base is tris(hydroxymethyl)aminomethane, and the fuchsin is new fuchsin.

In another aspect of the present disclosure, which however does not form part of the claimed invention, is a biological sample which has been stained with an acid fast staining composition that is free from phenol, the acid fast staining composition comprising a fuchsin, a base, a surfactant, and an alcohol. The biological sample may be one which has been stained with an acid fast staining composition consisting essentially of (a) new fuchsin in an amount ranging from between about 0.75 w/v% to about 2.75w/v% by total volume of the composition; (b) a weak base in an amount ranging from between about 0.05 w/v% to about 0.5 w/v% by total volume of the composition; and (c) a non-ionic surfactant in an amount ranging from between about 0.5w/v% to about 4w/v% by total volume of the composition.

In another aspect of the present invention is a kit comprising (a) an acid fast staining composition comprising a fuchsin, a weak base base having a pka ranging from about 8 to about 20, a non-ionic surfactant, and an alcohol, wherein an amount of fuchsin (e.g. new fuchsin) in the composition ranges from between about 0.75 w/v% to about 2.75 w/v% by total volume of the composition, wherein an amount of base in the composition ranges from between about 0.05 w/v% to about 0.5 w/v% by total volume of the composition, wherein , an amount of surfactant in the composition ranges from between about 0.5 w/v% to about 4 w/v% by total volume of the composition, and wherein the acid fast staining composition is free from phenol; and (b) a second composition selected from the group consisting of (i) a deparaffinization solution; (ii) a wash solution including a detergent; (iii) a decolorizing solution comprising a lower alcohol and an acid; and (iv) a secondary dye solution comprising a dye and a weak acid. In some embodiments, kit comprises (a) an acid fast staining composition comprising a fuchsin, a base, a surfactant, and an alcohol, wherein the acid fast composition is free from phenol; and at least two of additional compositions selected from the group consisting of (i) a deparaffinization solution; (ii) a wash solution including a detergent; (iii) a decolorizing solution comprising a lower alcohol and an acid; and (iv) a secondary dye solution comprising a dye and a weak acid. In some embodiments, the kit comprises a first container having an acid fast staining composition which is free from phenol; a second container having a decolorizing solution; and a third container having a secondary dye solution.

In another aspect of the present invention is an *in vitro* method of staining an acid fast organism in a biological sample (e.g. a histology sample, a cytology sample, etc.), the method comprising the steps of: (a) applying an acid fast staining composition of the present invention to a biological sample obtained from a subject having or suspected of having an infection with an acid fast organism; and (b) heating at least one of the acid fast staining composition or the biological sample to a temperature ranging from between about 30°C to about 45°C. In some embodiments, the sample is incubated with the acid fast staining composition for a time period ranging from between about 12 minutes to about 24 minutes. In some embodiments, between about 100 microliters to about 500 microliters of the acid fast staining composition is applied to the biological sample. In some embodiments, about 200 microliters of the acid fast staining composition is applied to the biological sample. In some embodiments, the method further comprises deparaffinizing the biological sample prior to applying the acid fast staining composition. In some embodiments, the method further comprises the steps of (d) applying a decolorizing solution including an alcohol and an acid to the biological sample; and (e) applying a secondary stain including a dye and a weak acid to the biological sample. In some embodiments, the method further comprises the step of imaging the biological sample. In some embodiments, the acid fast staining composition is applied with an automated staining system.

In another aspect of the present disclosure, which however does not form part of the claimed invention, is a method of detecting an acid fast organism in a biological sample (e.g. a histology sample, a cytology sample, etc.) comprising: (a) applying an acid fast staining solution to the biological sample, the acid fast staining solution comprising a fuchsin, a base, a surfactant, and an alcohol, wherein an amount of fuchsin in the composition ranges from between about 0.75 w/v% to about 2.75 w/v%; and (b) heating at least one of the staining solution or the biological sample to a temperature ranging from between about 30°C to about 45°C. In some instances, the staining solution has a pH ranging from between about 8 and about 20. In some instances, the sample is incubated with the staining solution for a time period ranging from between about 10 minutes to about 40 minutes. In some instances, the sample is incubated with the staining solution for a time period ranging from between about 12 minutes to about 24 minutes. In some instances, between about 100 microliters to about 500 microliters of staining solution is applied to the biological sample. In some instances, about 200 microliters of staining solution is applied to the biological sample.

In some instances, the method further comprises the step of deparaffinizing the biological sample prior to applying the staining solution. In some instances, the method further comprises the following steps after staining with the acid fast staining solution: (i) applying a decolorizing solution comprising an alcohol and an acid to the sample; and (ii) applying a secondary stain comprising a dye and a weak acid to the sample. In some instances, the method further comprises the step of imaging the biological sample. In some instances, the acid fast staining solution is applied to the biological sample with an automated specimen processing instrument.

In another aspect of the present disclosure, which however does not form part of the claimed invention, is a method of staining an acid fast organism in a biological sample disposed on a slide with an automated staining apparatus comprising: (a) loading the biological sample into the automated staining apparatus, (b) dispensing an acid fast staining composition onto the biological sample; and (c) removing the acid fast staining composition from the slide. In some instances, the biological sample is from a subject having or suspected of having an infection with an acid-fast organism. In some instances, between about 100 microliters to about 500 microliters of the acid fast staining composition is applied to the biological sample. In some instances, the biological sample is incubated with the acid fast staining composition for a period of time ranging from between about 10 minutes to about 30 minutes. In some instances, the biological sample is incubated at a temperature ranging from between about 30°C to about 45°C. In some instances, the method further comprises dispensing a decolorizing solution comprising an alcohol and an acid onto the sample. In some instances, the method further comprises dispensing a secondary stain composition comprising a dye and a weak acid onto the sample. In some instances, the method further comprises deparaffinizing the sample prior to application of the acid fast staining composition. In some instances, the method further comprises applying a coverslip to the sample disposed on the slide.

In another aspect of the present disclosure, which however does not form part of the claimed invention, is an apparatus comprising at least one dispenser configured to dispense an acid fast staining composition. In some embodiments, the apparatus further comprises at least one assembly adapted to heat the microscope slide.

In another aspect of the present disclosure, which however does not form part of the claimed invention, is a method of staining a sample disposed on a microscope slide with an automated staining apparatus comprising: (i) dispensing a phenol-free acid fast staining solution onto the slide, the staining solution comprising (a) fuchsin present in an amount ranging from between about 0.75 w/v% to about 2.75w/v% by total volume of the composition; (b) a base present in an amount ranging from between about 0.05 w/v% to about 0.5 w/v% by total volume of the composition; and (c) a surfactant present in an amount ranging from between about 0.5w/v% to about 4w/v% by total volume of the composition; and (ii) removing the acid fast staining solution from the slide after a predetermined amount of time. In some instances, between about 100 microliters to about 500 microliters of staining solution is applied to the biological sample. In some instances, about 200 microliters of staining solution is applied to the biological sample. In some instances, the specimen is incubated with the staining solution for a time period ranging from between about 10 minutes to about 40 minutes; and wherein at least one of the specimen or staining solution is heated to a temperature ranging from between about 30°C to about 45°C. In some instances, the specimen is heated to a temperature ranging from between about 30°C to about 45°C. In some instances, the sample is heated to a temperature ranging from between about 30°C to about 45°C. In some instances, both the sample and the specimen are heated to a temperature ranging from between about 30°C to about 45°C. In some instances, the method further comprises imaging the specimen (e.g. with a microscope or with a scanning device). In some instances, the method further comprises dispensing a first rinse fluid, a wash fluid, and/or buffer onto the slide.

In some instances, the method further comprises the steps of dispensing a decolorizing solution comprising an alcohol and an acid onto the slide; and removing the decolorizing solution from the slide. In some instances, the method further comprises dispensing a second rinse fluid or buffer onto the slide. In some instances, the method further comprises the steps of dispensing a secondary stain composition comprising a dye and a weak acid onto the slide; and removing the secondary stain composition. In some instances, the method further comprises dispensing a third rinse fluid or buffer to the slide.

In some instances, the method comprises deparaffinizing the sample prior to staining with the acid fast staining solution. In some instances, the method further comprises applying a coverslip over the sample. In some instances, the method further comprises analyzing the sample stained with the phenol-free acid fast staining solution to determine if an acid fast bacterium is present therein.

In another aspect of the present disclosure, which however does not form part of the claimed invention, is an apparatus comprising at least one dispenser configured to dispense an acid fast staining solution comprising a fuchsin, a base, a surfactant, and an alcohol onto a biological specimen, wherein the acid fast staining composition is free from phenol; and wherein the apparatus further comprises an assembly adapted to heat at least one of the biological specimen or the acid fast staining solution to a temperature of at least 30°C. In some instances, the base is tris(hydroxymethyl)aminomethane. In some instances, the surfactant is a non-ionic surfactant.

### BRIEF DESCRIPTION OF THE FIGURES

For a general understanding of the features of the disclosure, reference is made to the drawings. In the drawings, like reference numerals have been used throughout to identify identical elements.
FIG. 1 provides a graphical summary of slide reads for slides stained using a first instrument with an acid fast staining composition free from phenol.
FIG. 2 provides a graphical summary of slide reads for slides stained using a first instrument with an acid fast staining composition free from phenol.
FIG. 3 provides a graphical summary of slide reads for slides stained using two different instruments instrument, where the slides were stained with wither an acid fast staining composition free from phenol, or a traditional phenol-containing staining composition.
FIG. 4 provides a graph of pH for each design lot/condition at each of the test time points. Storage conditions are shown by the colored dots. The upper and lower failure limits are show as red gradient bars established by Day 0 measurements.
FIG. 5 sets forth a graph of design lot 1 HPLC measurements for each time point. Temperature conditions are indicated by the colored dots. The lower failure limit is show by a red line. All time points are above the failure limit.
FIG. 6 sets forth a graph of design lot 2 HPLC measurements for each time point. Temperature conditions are indicated by the colored dots. The lower failure limit is show by a red line. All time points are above the failure limit.
FIG. 7 sets forth a graph of design lot 2 3 HPLC measurements for each time point. Temperature conditions are indicated by the colored dots. The lower failure limit is show by a red line. All time points are above the failure limit.
FIG. 8 provides a graph of UV-Vis measurements for all DL at each time point. Temperature conditions are indicated by the colored dots. The upper and lower failure limits are shown as red gradient bars. All time points are passing.

### DETAILED DESCRIPTION

It should also be understood that, unless clearly indicated to the contrary, in any methods claimed herein that include more than one step or act, the order of the steps or acts of the method is not necessarily limited to the order in which the steps or acts of the method are recited.

As used herein, the singular terms "a," "an," and "the" include plural referents unless context clearly indicates otherwise. Similarly, the word "or" is intended to include "and" unless the context clearly indicates otherwise. The term "includes" is defined inclusively, such that "includes A or B" means including A, B, or A and B.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of" or "exactly one of." "Consisting essentially of," when used in the claims, shall have its ordinary meaning as used in the field of patent law.

The terms "comprising," "including," "having," and the like are used interchangeably and have the same meaning. Similarly, "comprises," "includes," "has," and the like are used interchangeably and have the same meaning. Specifically, each of the terms is defined consistent with the common United States patent law definition of "comprising" and is therefore interpreted to be an open term meaning "at least the following," and is also interpreted not to exclude additional features, limitations, aspects, etc. Thus, for example, "a device having components a, b, and c" means that the device includes at least components a, b and c. Similarly, the phrase: "a method involving steps a, b, and c" means that the method includes at least steps a, b, and c. Moreover, while the steps and processes may be outlined herein in a particular order, the skilled artisan will recognize that the ordering steps and processes may vary.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

As used herein, the term "acid fast" or "acid-fastness" refers to a physical property of certain bacterial and eukaryotic cells, as well as some subcellular structures, specifically their resistance to decolorization by acids during laboratory staining procedures. Once stained as part of a sample, these organisms can resist the acid and/or ethanol-based decolorization procedures common in many staining protocols.

As used herein, the term "acid-fast bacteria" or "AFB" refers to a bacterium that retains the stain after an acid wash. The term "AFB" used herein refers to acid-fast bacterium, and may include any bacterium having a character of being acid-fast. AFB, as used in the invention, may be of a Mycobacterium genus. The AFB may be of a genus other than Mycobacterium. Accordingly, the invention may be applied to AFB that is not a Mycobacterium. More specifically, the invention may be applied to AFB that is Corynebacterium, Propionibacterium, Tsukamurella or Actinomyces, Norcardium, to provide some examples.

As used herein, the term "biological sample" or "tissue sample" refers to any sample including a biomolecule (such as a protein, a peptide, a nucleic acid, a lipid, a carbohydrate, or a combination thereof) that is obtained from any organism including viruses. Other examples of organisms include mammals (such as humans; veterinary animals like cats, dogs, horses, cattle, and swine; and laboratory animals like mice, rats and primates), insects, annelids, arachnids, marsupials, reptiles, amphibians, bacteria, and fungi. Biological samples include tissue samples (such as tissue sections and needle biopsies of tissue), cell samples (such as cytological smears such as Pap smears or blood smears or samples of cells obtained by microdissection), or cell fractions, fragments or organelles (such as obtained by lysing cells and separating their components by centrifugation or otherwise). Other examples of biological samples include blood, serum, urine, semen, fecal matter, cerebrospinal fluid, interstitial fluid, mucous, tears, sweat, pus, biopsied tissue (for example, obtained by a surgical biopsy or a needle biopsy), nipple aspirates, cerumen, milk, vaginal fluid, saliva, swabs (such as buccal swabs), or any material containing biomolecules that is derived from a first biological sample. In certain embodiments, the term "biological sample" as used herein refers to a sample (such as a homogenized or liquefied sample) prepared from a tumor or a portion thereof obtained from a subject.

As used herein, "Cₐ to C_{b}" in which "a" and "b" are integers refer to the number of carbon atoms in an alkyl, alkenyl or alkynyl group, or the number of carbon atoms in the ring of a cycloalkyl, cycloalkenyl, cycloalkynyl or aryl group, or the total number of carbon atoms and heteroatoms in a heteroalkyl, heterocyclyl, heteroaryl or heteroalicyclyl group. That is, the alkyl, alkenyl, alkynyl, ring of the cycloalkyl, ring of the cycloalkenyl, ring of the cycloalkynyl, ring of the aryl, ring of the heteroaryl or ring of the heteroalicyclyl can contain from "a" to "b", inclusive, carbon atoms. Thus, for example, a "Ci to C₄ alkyl" group refers to all alkyl groups having from 1 to 4 carbons, that is, CH₃-, CH₃CH₂-, CH₃CH₂CH₂-, (CH₃)₂CH-, CH₃CH₂CH₂CH₂-, CH₃CH₂CH(CH₃)- and (CH₃)₃C-. If no "a" and "b" are designated with regard to an alkyl, alkenyl, alkynyl, cycloalkyl cycloalkenyl, cycloalkynyl, aryl, heteroaryl or heteroalicyclyl group, the broadest range described in these definitions is to be assumed.

As used herein, the term "fluid" refers to any liquid, including water, solvents, solutions (*e*.*g*. buffer solutions), etc. The term "fluids" also refers to any mixtures, colloids, suspensions, etc. The term "fluids" also encompasses reagents, stains, and other specimen processing agents (*e*.*g*. glues, fixatives, etc.) which may be applied to a microscope slide and/or specimen. The fluids may be aqueous or non-aqueous. Further examples include solutions or suspensions of antibodies, solutions or suspensions of nucleic acid probes, and solutions or suspensions of dye or stain molecules (e.g., H&E staining solutions, Pap staining solutions, etc.). Still further examples of fluids include solvents and/or solutions for deparaffinizing paraffin-embedded biological specimens, aqueous detergent solutions, and hydrocarbons (e.g., alkanes, isoalkanes and aromatic compounds such as xylene). Still further examples of fluids include solvents (and mixtures thereof) used to dehydrate or rehydrate biological specimens.

As used herein, the term "lower alcohol" refers to a C₁ - C₆ alcohol, which may be linear or branched. Examples of lower alcohols include methanol, ethanol, propanol, and butanol, as well as isomers thereof.

As used herein, the term "mycobacteria" is intended to encompass any known mycobacteria, including, but not limited to, Mycobacterium tuberculosis, Mycobacterium bovis, Mycobacterium microti, Mycobacterium africanum, Mycobacterium canetti, Mycobacterium avium, Mycobacterium intracellulare, Mycobacterium scrofulaceum, Mycobacterium kansasii, Mycobacterium malmoense, Mycobacterium xenopi, Mycobacterium marinum, Mycobacterium simiae, Mycobacterium terrae, Mycobacterium ulcerans, Mycobacterium abscessus, Mycobacterium fortuitum, Mycobacterium chelonae, and Mycobacterium gordonae.

As used herein, the term "reagent" may refer to any fluid deposited onto a tissue section or cytology sample, that is used in the context of a morphological (e.g. hematoxylin and eosin), immunohistochemical, or special stain. This includes, but is not limited to, oils, organics, and bridging reagents for removing wax (i.e. deparaffinization); washes, rinses, diluents, or buffers used to set reaction conditions, dilute reagents to an appropriate concentration, quench reactions, or wash away excess reactants; small molecule dyes used for morphological staining and special stains; antibodies, antibody conjugates, enzymes, multimers, amplifiers, chromogenic substrates, fluorescent detection chemistries, chemiluminescent substrates, and enzyme-reaction co-factors, used in IHC or ICC staining.

As used herein, the term "slide" refers to any substrate (e.g., substrates made, in whole or in part, glass, quartz, plastic, silicon, etc.) of any suitable dimensions on which a biological specimen is placed for analysis, and more particularly to a "microscope slide" such as a standard 3 inch by 1 inch microscope slide or a standard 75 mm by 25 mm microscope slide. Examples of biological specimens that can be placed on a slide include, without limitation, a cytological smear, a thin tissue section (such as from a biopsy), and an array of biological specimens, for example a tissue array, a cellular array, a DNA array, an RNA array, a protein array, or any combination thereof. Thus, in one embodiment, tissue sections, DNA samples, RNA samples, and/or proteins are placed on a slide at particular locations. In some embodiments, the term slide may refer to SELDI and MALDI chips, and silicon wafers.

As used herein, "surfactants" are classified as anionic, cationic, or non-ionic, depending on their mode of chemical action. In general, surfactants reduce interfacial tension between two liquids. A surfactant molecule typically has a polar or ionic "head" and a nonpolar hydrocarbon "tail." Upon dissolution in water, the surfactant molecules aggregate and form micelles, in which the nonpolar tails are oriented inward and the polar or ionic heads are oriented outward toward the aqueous environment. The nonpolar tails create a nonpolar "pocket" within the micelle. Nonpolar compounds in the solution are sequestered in the pockets formed by the surfactant molecules, thus allowing the nonpolar compounds to remain mixed within the aqueous solution. In some embodiments, the surfactant may be used to produce uniform spreading of reagents across a tissue section as well as decrease background staining.

As used herein, the terms "stain," "staining," or the like as used herein generally refers to any treatment of a biological specimen that detects and/or differentiates the presence, location, and/or amount (such as concentration) of a particular molecule (such as a lipid, protein or nucleic acid) or particular structure (such as a normal or malignant cell, cytosol, nucleus, Golgi apparatus, or cytoskeleton) in the biological specimen. For example, staining can provide contrast between a particular molecule or a particular cellular structure and surrounding portions of a biological specimen, and the intensity of the staining can provide a measure of the amount of a particular molecule in the specimen. Staining can be used to aid in the viewing of molecules, cellular structures and organisms not only with bright-field microscopes, but also with other viewing tools, such as phase contrast microscopes, electron microscopes, and fluorescence microscopes. Some staining performed by the system 2 can be used to visualize an outline of a cell. Other staining performed by the system 2 may rely on certain cell components (such as molecules or structures) being stained without or with relatively little staining other cell components. Examples of types of staining methods performed by the system 2 include, without limitation, histochemical methods, immunohistochemical methods, and other methods based on reactions between molecules (including non-covalent binding interactions), such as hybridization reactions between nucleic acid molecules. Particular staining methods include, but are not limited to, primary staining methods (e.g., H&E staining, Pap staining, etc.), enzyme-linked immunohistochemical methods, and in situ RNA and DNA hybridization methods, such as fluorescence in situ hybridization (FISH).

As used herein, a "Ziehl-Neelsen stain" means a stain used in the demonstration of acid fast bacteria belonging to the genus mycobacterium, which include the causative agent for tuberculosis.

### Overview

The present disclosure relates to acid fast staining compositions which are free from phenol. The present disclosure also provides methods of applying those compositions to biological samples to enable detection of an acid fast bacterium present therein.

### Acid fast staining compositions

In one aspect of the present invention is an acid fast staining composition comprising a fuchsin, a weak base having a pka ranging from about 8 to about 20, a non-ionic surfactant, and an alcohol, wherein an amount of fuchsin (e.g. new fuchsin) in the composition ranges from between about 0.75 w/v% to about 2.75 w/v% by total volume of the composition, wherein an amount of base in the composition ranges from between about 0.05 w/v% to about 0.5 w/v% by total volume of the composition, wherein , an amount of surfactant in the composition ranges from between about 0.5 w/v% to about 4 w/v% by total volume of the composition, and wherein the composition is free from phenol.

In some embodiments, a single fuchsin is included within any acid fast staining composition. Examples of fuchsin compounds include new fuchsin, acid fuchsin, basic fuchsin, and derivatives or analogs thereof. In some embodiments, the fuchsin is new fuchsin.

In some embodiments, the amount of fuchsin in the composition ranges from 0.75 w/v% to about 2.5 w/v% by total volume of the composition. In further embodiments, the amount of fuchsin in the composition ranges from 1 w/v% to about 2.5 w/v% by total volume of the composition. In even further embodiments, the amount of fuchsin in the composition ranges from 1.25 w/v% to about 2.5 w/v% by total volume of the composition. In yet even further embodiments, the amount of fuchsin in the composition ranges from 1 w/v% to about 2.25 w/v% by total volume of the composition.

In some embodiments, the base is a hydroxide. In some embodiments, the base is a non-nucleophilic base. In some embodiments, the base has a pH ranging from between about 9 and about 11. Suitable weak bases include tris(hydroxymethyl)aminomethane ("TRIS"), ammonia, pyridine, organic amines (e.g. trimethylamine), carboxylates, carbonates. In other embodiments, the base is TRIS. In some embodiments, the base is selected from one which does not react with the fuchsin to form an ethoxylated fuchsin derivative.

In some embodiments, the amount of base ranges from between about 0.075 w/v% to about 0.45 w/v% by total volume of the composition. In other embodiments, the amount of base ranges from between about 0.1 w/v% to about 0.4 w/v% by total volume of the composition. In other embodiments, the amount of base ranges from between about 0.1 w/v% to about 0.35 w/v% by total volume of the composition. In yet other embodiments, the amount of base ranges from between about 0.125 w/v% to about 0.3 w/v% by total volume of the composition. In yet other embodiments, the amount of base ranges from between about 0.1 w/v% to about 0.2 w/v% by total volume of the composition. In yet other embodiments, the amount of base ranges from between about 0.2 w/v% to about 0.45 w/v% by total volume of the composition.

Among the suitable non-ionic surfactants are condensation products of C₈ - C₃₀ alcohols with sugar or starch polymers. These compounds can be represented by the formula (S)ₙ -O-R, wherein S is a sugar moiety such as glucose, fructose, mannose, and galactose; n is an integer of from about 1 to about 1000, and R is C₈ - C₃₀ alkyl. Examples of suitable C₈ - C₃₀ alcohols from which the R group may be derived include decyl alcohol, cetyl alcohol, stearyl alcohol, lauryl alcohol, myristyl alcohol, oleyl alcohol, and the like. Specific examples of these surfactants include decyl polyglucoside and lauryl polyglucoside.

Other suitable non-ionic surfactants include the condensation products of alkylene oxides with fatty acids (i.e., alkylene oxide esters of fatty acids). These materials have the general formula RCO(X)ₙ OH, wherein R is a C₁₀-C₃₀ alkyl, X is -OCH₂CH₂- (derived from ethylene oxide) or -OCH₂CHCH₃-(derived from propylene oxide), and n is an integer from about 1 to about 200.

Yet other suitable non-ionic surfactants are the condensation products of alkylene oxides with fatty acids (i.e., alkylene oxide diesters of fatty acids) having the formula RCO(X)ₙOOCR, wherein R is a C₁₀ - C₃₀ alkyl, X is -OCH₂CH₂-(derived from ethylene oxide) or -OCH₂CHCH₃- (derived from propylene oxide), and n is an integer from about 1 to about 200. Yet other non-ionic surfactants are the condensation products of alkylene oxides with fatty alcohols (i.e., alkylene oxide ethers of fatty alcohols) having the general formula R(X)ₙOR', wherein R is C₁₀-C₃₀ alkyl, n is an integer from about 1 to about 200, and R' is H or a C₁₀-C₃₀ alkyl.

Still other non-ionic surfactants are the compounds having the formula RCO(X)ₙOR' wherein R and R' are C₁₀-C₃₀ alkyl, X is -OCH₂CH₂-(derived from ethylene oxide) or -OCH₂CHCH₃- (derived from propylene oxide), and n is an integer from about 1 to about 200. Examples of alkylene oxide-derived non-ionic surfactants include ceteth-1, ceteth-2, ceteth-6, ceteth-10, ceteth-12, ceteraeth-2, ceteareth6, ceteareth-10, ceteareth-12, steareth-1, steareth-2, stearteth-6, steareth-10, steareth-12, PEG-2 stearate, PEG4 stearate, PEG6 stearate, PEG-10 stearate, PEG-12 stearate, PEG-20 glyceryl stearate, PEG-80 glyceryl tallowate, PPG-10 glyceryl stearate, PEG-30 glyceryl cocoate, PEG-80 glyceryl cocoate, PEG-200 glyceryl tallowate, PEG-8 dilaurate, PEG-10 distearate, and mixtures thereof. Still other useful non-ionic surfactants include polyhydroxy fatty acid amides disclosed, for example, in U.S. Pat. Nos. 2,965,576, 2,703,798, and 1,985,424.

Examples of non-ionic surfactants that are generally known to the art and to the literature include various linear ethoxylates such as primary or secondary alcohol ethoxylates, other alcohol alkoxylates, aromatic ethoxylates, modified ethoxylates, and blends thereof. Examples include but are not limited to, C8-C18 alcohol ethoxylates, including those C8-C18 alcohol ethoxylates with less than 12 moles of ethylene oxide (EO). Exemplary surfactants include Tomadol 1200 (Air Products), Tomadol 900 (Air Products), Tomadol 91-8 (Air Products), Tomadol 1-9 (Air Products), Tergitol 15-S-9 (Sigma), Tergitol 15-S-12 (Sigma), Masurf NRW-N (Pilot Chemical), Bio-Soft N91-6 (Stepan), and Brij-35 (Polyethylene glycol dodecyl ether) (Sigma). As used herein, "Tergitol" surfactants are defined by the following formula, where n, n1 and n2 are independently 5-30. For Tergitol 15-S-5, for example, n+n1=12, n2=4; for Tergitol 15-S-7, n+n1=12 and n2=6; and for Tergitol 15-S-9, n+n1=12 and n2=8. Further examples of polyoxyethylene include Nonoxynol-9, Nonidet P-40, and Igepal series surfactants although many others are known.

Examples of other non-ionic surfactants include copolymers of poly(ethylene oxide) and polypropylene oxide) (e.g., poloxamers, such as BASF PLURONIC^{®} products). Further examples of non-ionic surfactants include, but are not limited to, 8-methyl-1-nonanol propoxylate-block-ethoxylate, ALKANOL^{®} 6112, allyl alcohol 1,2-butoxylate-block-ethoxylate, Brij@ 30, Brij@ 52, Brij@ 72, Brij@ 78, Brij@ 92V, Brij@ 93, Brij@ 97, Brij@ 98, Brij@ 010, Brij@ 5100, Brij@ 510, Brij@ 58, IGEPAL^{®} CA-210, IGEPAL^{®} CA-520, IGEPAL^{®} CA-720, IGEPAL^{®} CO-210, IGEPAL^{®} CO-520, IGEPAL^{®} CO-630, IGEPAL^{®} CO-720, IGEPAL^{®} CO-890, IGEPAL^{®} DM-970, MERPOL@ A, MERPOL@ DA, MERPOL@ HCS, MERPOL@ OJ, MERPOL@ SE, MERPOL@ SH, polyethylene-block-poly(ethylene glycol), polyoxyethylene tridecyl ether, polyoxyethylene sorbitan tetraoleate, polyoxyethylene sorbitol hexaoleate, sorbitan monopalmitate, TWEEN^{®} 20, TWEEN^{®} 40, TWEEN^{®} 60, TWEEN^{®} 85.

In some embodiments, an amount of surfactant in the composition ranges from between about 0.75 w/v% to about 3.5 w/v% by total volume of the composition. In other embodiments, an amount of surfactant in the composition ranges from between about 1 w/v% to about 3 w/v% by total volume of the composition. In further embodiments, an amount of surfactant in the composition ranges from between about 1.5 w/v% to about 2.5 w/v% by total volume of the composition.

In some embodiments, the alcohol is a lower alcohol, which may be branched or straight chain. In some embodiments, the alcohol is ethanol, n-propanol, isopropanol, or mixtures thereto.

In some embodiments, the acid fast staining composition comprises new fuchsin, a non-ionic surfactant, and TRIS, where (a) the new fuchsin is present in an amount ranging from between about 0.75 w/v% to about 2.75w/v% by total volume of the composition; (b) TRIS is present in an amount ranging from between about 0.1 w/v% to about 0.5 w/v% by total volume of the composition; and (c) the non-ionic surfactant is present in an amount ranging from between about 0.5w/v% to about 4w/v% by total volume of the composition.

In some embodiments, the acid fast staining composition is stable for at least 120 days. In some embodiments, the acid fast staining composition is stable for at least 180 days. In some embodiments, the acid fast staining composition is stable for at least 240 days. In some embodiments, the acid fast staining composition is stable for at least 300 days. In some embodiments, the acid fast staining composition is stable for at least 360 days. In some embodiments, the acid fast staining composition is stable for at least 420 days. In some embodiments, the acid fast staining composition is stable for at least 450 days. In some embodiments, the acid fast staining composition is stable for at least 500 days. In some embodiments, the acid fast staining composition is stable for at least 520 days. In some embodiments, the acid fast staining composition is stable for at least 540 days. In some embodiments, the acid fast staining composition is stable for at least 580 days. In some embodiments, the acid fast staining composition is stable for at least 600 days. In some embodiments, the acid fast staining composition is stable for at least 620 days. In some embodiments, the acid fast staining composition is stable for at least 630 days.

Non-limiting examples of acid fast staining compositions are set forth in Table 1:

| Composition Number | Component Varied (Concentration) | New Fuchsin w/v % | Surfactant w/v % | Base w/v % | Solvent and Total Volume |
|---|---|---|---|---|---|
| 1 | Fuchsin | 1.25 | 2.0 | 0.15 | Alcohol 150 mL Each |
| 2 | Fuchsin | 1.5 | 2.0 | 0.15 | |
| 3 | Fuchsin | 2.0 | 2.0 | 0.15 | |
| 4 | Fuchsin | 2.25 | 2.0 | 0.15 | |
| 5 | Tergitol 15-S-9 | 1.75 | 1.0 | 0.15 | |
| 6 | Tergitol 15-S-9 | 1.75 | 1.5 | 0.15 | |
| 7 | Tergitol 15-S-9 | 1.75 | 2.5 | 0.15 | |
| 8 | Tergitol 15-S-9 | 1.75 | 3.0 | 0.15 | |
| 9 | KOH | 1.75 | 2.0 | 0.1 | |
| 10 | KOH | 1.75 | 2.0 | 0.125 | |
| 11 | KOH | 1.75 | 2.0 | 0.175 | |
| 12 | KOH | 1.75 | 2.0 | 0.2 | |
| 13 | TRIS | 1.75 | 2.0 | 0.22 | |
| 14 | TRIS | 1.75 | 2.0 | 0.32 | |
| 15 | TRIS | 1.75 | 2.0 | 0.43 | |

### Methods

In another aspect of the present invention is an *in vitro* method of staining an acid fast organism (e.g. mycobacterium) in a biological sample (e.g. a histology sample, a cytology sample, etc.), the method comprising (a) applying an acid fast staining composition according to the present disclosure to a biological sample obtained from a subject having or suspected of having an infection with an acid fast organism, and (b) heating at least one of the acid fast staining composition or the biological sample to a temperature ranging from between about 30°C to about 45°C.

In some embodiments, the acid fast staining composition comprises new fuchsin, a non-ionic surfactant, and TRIS where (a) the new fuchsin is present in an amount ranging from between about 0.75 w/v% to about 2.75w/v% by total volume of the composition; (b) TRIS is present in an amount ranging from between about 0.1 w/v% to about 0.5 w/v% by total volume of the composition; and (c) the non-ionic surfactant is present in an amount ranging from between about 0.5w/v% to about 4w/v% by total volume of the composition. Of course, any of the acid fast staining compositions noted herein, including those identified in Table 1, may be applied to a biological sample to effectuate detection of an acid fast organism in the sample. The step of applying the acid fast staining composition to the biological sample can be performed manually or with a specimen processing apparatus, as noted herein.

In some embodiments, at least one of the biological sample or the acid fast staining composition is heated to a temperature ranging from between about 35°C to about 40°C. In some embodiments, at least one of the biological sample or the acid fast staining composition is heated to a temperature of about 37°C.

In some embodiments, at least one of the biological sample or the acid fast staining composition is heated for a time period ranging from between about 10 minutes to about 40 minutes. In other embodiments, at least one of the biological sample or the acid fast staining composition is heated for a time period ranging from between about 15 minutes to about 40 minutes. In yet other embodiments, at least one of the biological sample or the acid fast staining composition is heated for a time period ranging from between about 15 minutes to about 30 minutes. In further embodiments, at least one of the biological sample or the acid fast staining composition is heated for a time period ranging from between about 12 minutes to about 24 minutes. In yet further embodiments, at least one of the biological sample or the acid fast staining composition is heated for a time period ranging from between about 16 minutes to about 36 minutes. In one embodiment, at least one of the biological sample or the acid fast staining composition is heated for about 12 minutes. In one embodiment, at least one of the biological sample or the acid fast staining composition is heated for about 16 minutes. In one embodiment, at least one of the biological sample or the acid fast staining composition is heated for about 20 minutes.

In some embodiments, the biological sample is incubated with the acid fast staining composition for an additional time period after heating, e.g. a time period in which the biological sample is allowed to cool. In some embodiments, this additional time period ranges from between about 1 minute to about 15 minutes. In other embodiments, this additional time period ranges from about 5 minutes to about 10 minutes. In some embodiments, a total time in which the composition remains in contact with the sample ranges from about 5 minutes to about 80 minutes.

In some embodiments, the methods further comprise contacting the biological sample with other fluids and/or reagents. For example, in some embodiments, additional stains, including counterstains, may be applied to the biological sample. In other embodiment, wash reagents, detection reagents, deparaffinization reagents, buffers, etc. may be applied prior to or subsequent to the deposition of the acid fast staining composition.

### Automated Specimen Processing Systems

The acid fast staining compositions of the present invention may be deposited using a specimen processing system. In some embodiments, a specimen processing apparatus is an automated apparatus, such as the BENCHMARK XT instrument, the BenchMark Special Stains instrument, the NexES Special Stainer instrument, the SYMPHONY instrument, or the BENCHMARK ULTRA instrument sold by Ventana Medical Systems, Inc. Ventana Medical Systems, Inc. is the assignee of a number of United States patents disclosing systems and methods for performing automated analyses, including U.S. Pat. Nos. 5,650,327, 5,654,200, 6,296,809, 6,352,861, 6,827,901 and 6,943,029, and U.S. Published Patent Application Nos. 2003/0211630 and 2004/0052685. Alternatively, specimens can be manually processed.

Examples of other commercially available specimen processing systems through which the acid fast staining composition may be applied include the VENTANA SYMPHONY (individual slide stainer) and the VENTANA HE 600 (individual slide stainer) series; the Dako CoverStainer (batch stainer) from Agilent Technologies; the Leica ST4020 Small Linear Stainer (batch stainer), Leica ST5020 Multistainer (batch stainer), and the Leica ST5010 Autostainer XL series (batch stainer) H&E stainers from Leica Biosystems Nussloch GmbH.

In some embodiments, the staining system may be adapted to perform all or some of the steps of processing, staining and coverslipping of substrate (e.g. slide) mounted specimens. In some embodiments, slides bearing biological specimens are placed on a slide tray, and the slide tray bearing the sample slides are loaded into the system where the slides are conducted through a sequence of steps in which the slides are baked, de-waxed, stained and finally coverslipped. In some embodiments, the methods disclosed herein are directed to a method of automatically preparing tissue samples on microscope slides (or other substrates) for pathological analysis, comprising baking the tissue sample onto the slide by having the instrument apply heat to the tissue sufficient to adhere it to the slide; deparaffinizing the tissue sample by contacting it with deparaffinizing fluid at a temperature above the melting point of the paraffin, and subsequently rinsing the liquefied paraffin away; staining the tissue sample by contacting it with a staining reagent; and coverslipping the slide by contacting the stained tissue sample on the slide with a pre-glued coverslip and an adhesive activating fluid. In some embodiments, the methods disclosed herein utilize only some of the aforementioned steps.

The specimen processing apparatus may include a carousel for holding a plurality of substrates, e.g. microscope slides, wherein each substrate includes a biological sample to be stained. The automatic staining equipment can also include a device for rotating the carousel at predetermined speeds and a mechanism for directing and controlling application of reagents, including the acid fast staining compositions, onto the substrates and samples during rotation of the carousel. Once the slides are loaded into the instrument, test protocols will dictate which reagents are dispensed onto the substrates at specific times. At the appropriate time, a dispenser rack will rotate to align a correct reagent over a substrate and the instrument will dispense a predetermined amount of a reagent or fluid onto the substrate (e.g. an acid fast staining composition which is free from phenol).

In some embodiments, the system is an automated slide processing system that includes a slide tray holding a plurality of slides in a substantially horizontal position (such as in two rows where the slides are held at an angle between about 0.2 degrees and about 1.2 degrees from horizontal) and one or more workstations (for example, arranged in a vertical stack) that receive the slide tray and perform one or more slide processing operations on slides in the slide tray. In some embodiments, the workstation can perform a slide processing operation on one or more individual slides in a slide tray, for example, at least two or four slides in a slide tray, or it can simultaneously perform a slide processing operation on all of the slides in a slide tray. In some embodiments, the one or more workstations dispense a reagent to slides in the slide tray without a substantial amount of the reagent that contacts a first slide contacting a second slide, thereby minimizing cross-contamination between slides. Such workstations can include one or more directional nozzles that dispense the reagent onto the slides, for example, the one or more directional nozzles can include a pair of directional nozzles that dispense the reagent in opposite directions across a surface of a slide. In more particular embodiments, the one or more directional nozzles can further include a directional nozzle that dispenses the reagent towards a bottom surface of a slide. In other particular embodiments, the one or more workstations can simultaneously dispense a reagent (for example, the same reagent) to at least two slides held in a slide tray within a given workstation, or the one or more workstations can simultaneously dispense a reagent (such as the same reagent) to all of the slides held in the slide tray within a given workstation. Additional system components and tray configurations (as well as control systems) are described in United States Patent Nos. 8,663,991, 7,468,161, and 9,528,918.

In some embodiments, the specimen processing apparatus is configured to dispense a predetermined amount of an acid fast staining composition to a biological sample. In some embodiments, the specimen processing apparatus is configured to dispense at least about 100 microliters of the acid fast staining composition to a biological sample. In other embodiments, at least about 150 microliters of the acid fast staining composition is dispensed to the biological sample. In yet other embodiments, at least about 200 microliters of the acid fast staining composition is dispensed to the biological sample. In further embodiments, between about 200 microliters and about 500 microliters of the acid fast staining composition is dispensed to the biological sample.

In some embodiments, the automated specimen processing apparatus includes a heating or cooling device (such as a conductive heater or a Peltier device) such that at least one of the biological sample or the acid fast staining composition is heated to a predetermined temperature and/or for a predetermined amount of time. Suitable examples of slide heating devices are described in US Patent Nos. 7,425,306 and 6,582,962. In some embodiments, at least one of the sample or the acid fast staining composition is heated to a temperature ranging from between about 30°C to about 45°C. In other embodiments, the specimen processing apparatus heats at least one of the biological sample or the acid fast staining composition to a temperature ranging from between about 35°C to about 40°C. In some embodiments, the specimen processing apparatus may heat the sample or the staining composition for a period of time as noted herein, e.g. for between 10 minutes and 40 minutes. In one embodiment, the specimen processing apparatus incubates the biological sample or the acid fast staining composition for about 12 minutes. In another embodiment, the specimen processing apparatus incubates the biological sample or the acid fast staining composition for about 16 minutes. In yet another embodiment, the specimen processing apparatus incubates the biological sample or the acid fast staining composition for about 20 minutes.

In some embodiments, the specimen is subsequently allowed to cool prior to further processing, e.g. removing or washing the acid fast staining composition from the slide. In some embodiments, the slide processing apparatus may provide for a "cool down" time ranging from between about 1 minutes to about 20 minutes. In some embodiments, the cool down time is about 8 minutes in duration.

The specimen processing apparatus may also be configured to apply other fluids and/or reagents both before and after dispensing of the acid fast staining composition to the biological sample. Indeed, the specimen processing apparatus can apply a wide range of substances to the specimen including, without limitation, stains, probes, reagents, rinses, and/or conditioners, or any of the other fluids and/or reagents recited herein. Probes can be an isolated nucleic acid or an isolated synthetic oligonucleotide, attached to a detectable label. Labels can include radioactive isotopes, enzyme substrates, co-factors, ligands, chemiluminescent or fluorescent agents, haptens, and enzymes. In some embodiments, the specimen processing apparatus facilitates performing an immunoassay, for example by incubation with one or more antibodies specific for a particular target bacterium and detected using a label (such as a label on the antibody or via use of a labeled secondary antibody). Exemplary detectable labels include fluorophores, haptens, enzymes, radiolabels, and others known in the art.

The specimen processing apparatus may be further adapted to apply a decolorizing solution a secondary stain to the biological sample. In some embodiments, a decolorizing solution may comprise an alcohol and an acid. Suitable decolorizing compositions and methods of their application (both manual and through use of an automated specimen processing apparatus) are disclosed in United States Patent No. 9,023,615. In some embodiments, the specimen processing apparatus may apply a secondary stain comprising a dye and a weak acid. In some embodiments, between about 200 microliters and about 500 microliters of either the decolorizing solution and/or the secondary stain is dispensed to the biological sample.

In some embodiments, the specimen processing apparatus facilitates other microbiological assays. For example, the sample can be contacted with one or more other dyes, such as one or more of the following stains: Alcian Blue, Alcian Blue for Periodic Acid Schiff (PAS), Alcian Yellow, Congo Red, Diastase, Elastic, Giemsa, Grocott's Methenamine Silver stain (GMS) II, iron, Jones Light Green, Jones, Light Green for PAS, Mucicamine PAS, Reticulum, Steiner II, Trichrome Blue, and Trichrome Green.

In some embodiments, if the specimen is a sample embedded in paraffin, the sample can be deparaffinized with the specimen processing apparatus using appropriate deparaffinizing fluid(s). In some embodiments, and after a waste remover device of a specimen processing apparatus removes any deparaffinizing fluid(s), any number of substances can be successively applied to the specimen. The substances can be for pretreatment (e.g., protein-crosslinking, expose nucleic acids, etc.), denaturation, hybridization, washing (e.g., stringency wash), detection (e.g., link a visual or marker molecule to a probe), amplifying (e.g., amplifying proteins, genes, etc.), counterstaining, coverslipping, or the like.

Analysis of a biological specimen stained in accordance with the procedures described herein can be automated, and facilitated by a computer analysis and/or image analysis system. In some embodiments, light microscopy is utilized for image analysis. Certain disclosed embodiments involve acquiring digital images. This can be done by coupling a digital camera to a microscope (e.g. a brightfield microscope). Digital images obtained of stained samples are analyzed using image analysis software. The samples also can be evaluated qualitatively and semi-quantitatively. Qualitative assessment includes assessing the staining intensity, identifying the positively-staining cells and the intracellular compartments involved in staining, and evaluating the overall sample or slide quality. Separate evaluations are performed on the test samples and this analysis can include a comparison to known average values to determine if the samples represent an abnormal state.

### Kits

The present invention also provides kits including (a) an acid fast staining composition of the present invention, and (b) a second composition selected from the group consisting of (I) a deparaffinization solution; (ii) a wash solution including a detergent; (iii) a decolorizing solution including a lower alcohol and an acid; and (iv) a secondary dye solution including a dye and a weak acid. In some embodiments, a kit includes the acid fast staining composition of the present invention and at least one of a decolorizing solution (e.g. AFB Decolorizer II, available from Ventana Medical Systems, Inc., Tucson, AZ) or a secondary dye solution (e.g. AFB III Blue, available from Ventana Medical Systems, Inc., Tucson, AZ). In yet other embodiments, a kit includes the acid fast staining composition of the present invention, decolorizing solution, and secondary dye solution. In some embodiments, each component of the kit is maintained in a separate container. In an embodiment, the kit comprises the acid fast staining composition of the present invention as a first composition, and further comprises the decolorizing solution and the secondary dye solution, each in separate containers

In some embodiments, the acid fast staining compositions are provided in a container. In some embodiments, the container is configured to be used with a specimen processing apparatus. For example, the container may be one that is adapted for use with Ventana Medical Systems, Inc. equipment such as the NexES Special Stainer ("NesES SS") or BenchMark Special Stainer ("BKMKSS"). In some embodiments, the acid fast staining compositions are provided in a dispenser for use in a specimen processing apparatus.

In some embodiments, the kits may also include one or more microscope slides, such as a slide suitable for mounting and in some examples fixing a sample, as well as coverslips, pipettes, or combinations thereof. In some embodiments, the kit can optionally further include additional reagents for performing additional assays. For example, the kit can include one or more vessels or containers that contain other dyes, stains, or counterstains, such as containers that include one or more of the following stains: Alcian Blue, Alcian Blue for Periodic Acid Schiff (PAS), Alcian Yellow, Congo Red, Diastase, Elastic, Giemsa, Grocott's Methenamine Silver stain (GMS) II, iron, Jones Light Green, Jones, Light Green for PAS, Mucicamine PAS, Reticulum, Steiner II, Trichrome Blue, and Trichrome Green. In one example, the kit can include a vessel or container that contains bacteria-specific antibodies. In some examples, the kit includes a vessel or container that contains labeled secondary antibodies (e.g., labeled with a fluorophore). In some embodiments, the kit comprises instructions for detecting an acid fast bacterium in a biological sample. In other embodiments, the kit comprises instructions for use of the acid fast staining composition in conjunction with a specimen processing apparatus.

### EXAMPLES

### Example 1 - Staining Composition Comparison Study

A study was developed to test the presently disclosed acid fast staining compositions against traditional phenol-containing acid fast staining compositions. This study was designed to demonstrate that acid fast staining compositions prepared over a range TRIS base concentrations, including 0.22 w/v% ("low TRIS"), 0.32% w/v% ("nominal TRIS"), and 0.43 w/v% ("high TRIS), result in functional staining that was at least comparable to staining using a phenol-containing acid fast staining composition (e.g. Ventana AFB III, available from Ventana Medical Systems, Inc., Tucson, Arizona, USA) (hereinafter "AFB III").

Three tissue cases exhibiting varying degrees of AFB microbe infection were selected for this study. For each case, three sets of near-cut slides (6 slides per case = 18 total slides per run) were used to assess pathologist preference for one slide vs the other in blinded pairs. Slides were stained either with AFB III or one of the compositions. Stained slides were dehydrated through two rinses each of 95% reagent alcohol, 100% reagent alcohol, and 100% xylene. The slides were coverslipped. All slides were visualized in pairs, e.g. a slide pair having slides stained with "low TRIS" and "nominal TRIS," or a slide pair having slides staining with "nominal TRIS" and "high TRIS." Determination of "PASS"/"FAIL" was determined by a count of total number of slides that passed/failed within each run on different specimen processing systems, namely Benchmark Special Stains automated stainer (BMKSS) and NexES Special Stainer (available from Ventana Medical Systems, Inc. Tucson, AZ), the PASS/FAIL determined by deciding whether one slide in a slide pair was preferred or not preferred relative to the other slide in the pair.

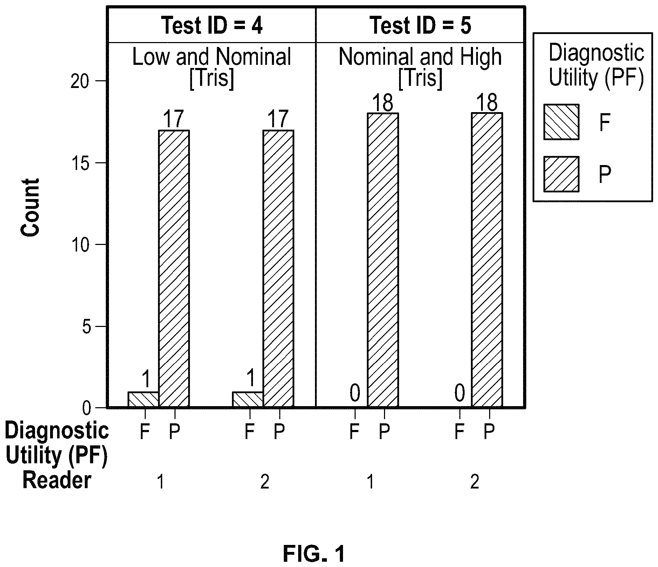

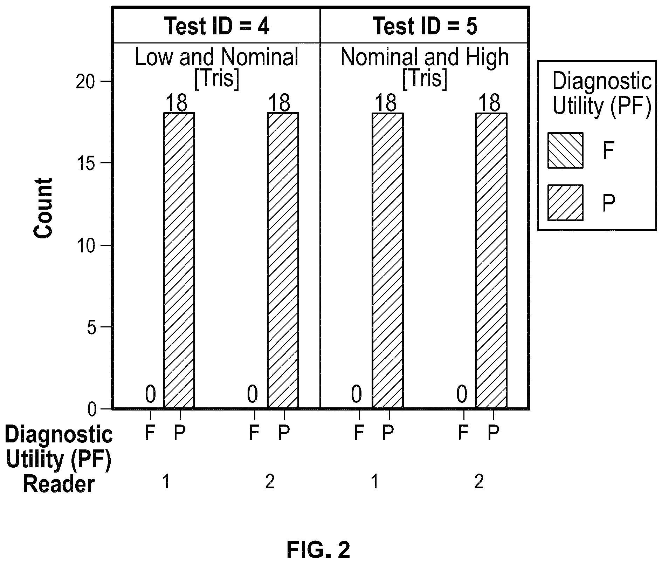

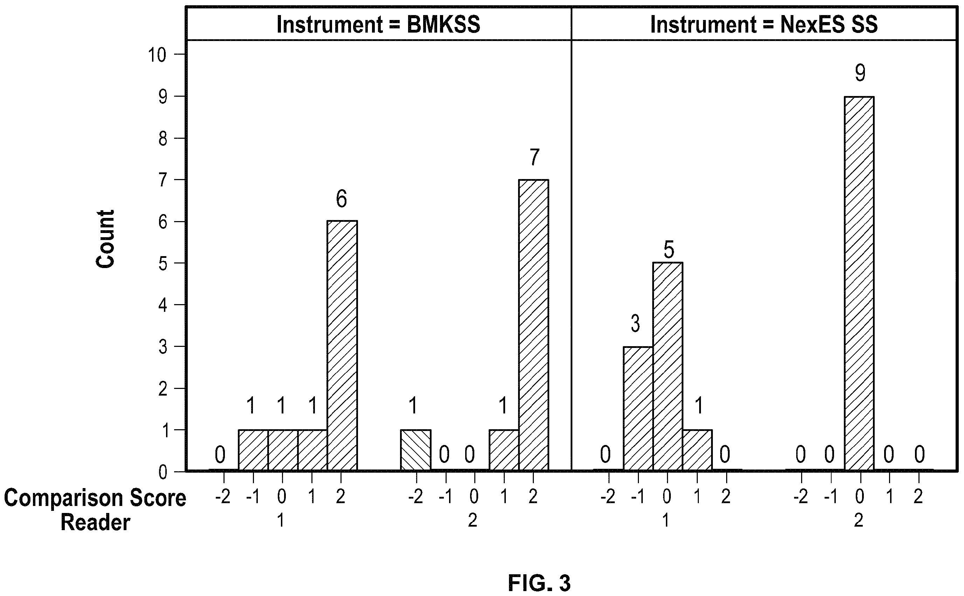

For BMKSS, each pathologist passed 18/18 slides per for the high TRIS - nominal TRIS (i.e. 0.32w/v%) pair, while passing 17/18 slides each for the low TRIS - nominal TRIS pairs (see FIG. 1). The single failed slide appeared to be the result of decolorizer not coming into contact with the needle-biopsy-sized tissue, according to pathologist comments, and not due to a failure of the phenol-free stain composition itself. For NexES SS, all of the slides passed (see FIG. 2). Pathologists also evaluated slides stained with "nominal TRIS" and those stained with AFB III. As illustrated in FIG. 3, pathologists preferred the slides stained with "nominal TRIS."

### Example 2 - Accelerated Stability Study

A study was developed to establish the initial product dating (IPD) of the new acid fast staining compositions under accelerated conditions at 60°C using three DLs that were prepared using each of the available vendor lot raw ingredients. The study did not vary individual acid fast staining composition component concentrations.

### MATERIALS

Tissue Requirements: Each tissue block used in this study was qualified for AFB-positivity, with a total of 54 - 100 slides (number dependent on the specific block, including excess cuts) to be cut from each block. A total of 183 slides were stained, including reruns.

Tissue Cut Bracketing: The first and every 25th slide of each block was stained with on-market AFB stain kit to determine eligibility for enrollment in the upcoming time point. All slides showed presence of AFB-positive microorganisms and passed. Day 0 slides were then stained and evaluated. After reading the Day 0 slides, it was decided to remove Block 3 from the study due to staining inconsistencies and tissue quality problems. Block 3 was replaced by Block 5, which was intended as a reserve in the event that such inconsistencies were discovered within the primary blocks (1-4).

Controls: The baseline controls used for paired comparisons at each of the specified weekly time points were prepared on Day 0 of the study. This required a total of 45 slides to be stained as controls. Nine additional Day 0 slides had to be stained after Day 0, however, as it was later identified that they had received the wrong design lot due to a labeling issue. The appropriate references were stained with the correct design lot and used as intended. The use of the new reference slides was appropriate since analytical testing and functional staining using the solution stored under ambient conditions showed no changes to the performance or composition of the AFB stain over the course of the study up to the time the mistake was discovered (1 month).

Bulk Reagents: The following bulk reagent materials were used. There were no lot restrictions for these reagents.
BenchMark Special Stains Liquid Coverslip, PIN 860-034
BenchMark Special Stains Two Part Wash Kit, PIN 860-040
BenchMark Special Stains Deparaffinization Solution (10X), PIN 860-036

### EQUIPMENT

Hardware/Software: A single, properly maintained BMKSS instrument was used for all functional staining in this study.

Environmental Chamber Temperature: An appropriate sensor within calibration was used to track and record the actual temperature during testing. All temperature conditions were maintained within the excursion of 5°C. AFB Stain design lots stored at the maximum temperature condition, 60°C, which was maintained over the course of this study, were used to establish initial product dating. The other temperature conditions were included in this study to ensure proper performance over a wide range of conditions, but not used to establish product dating.

### ACCELERATED STABILITY DESIGN

When using the Arrhenius model, it is assumed that reagent degradation is a simple process that is largely governed by first order kinetics over the temperature range considered. Also, the Q rule is useful to identify appropriate temperatures to use for accelerated stability studies (see Anderson & Scott, Clin. Chem. 1991, 37, 398). The Q rule states that a product degradation rate changes by a constant factor when storage temperature is changed by 10°C (reaction rate approximately doubles for each 10 C increase in temperature). The value of Q was typically set at 2, 3, or 4. A Q value of 2 is the most conservative model, which was used for this study (see Table 3).

A "test condition" was defined as a specific design lot solution under a specific stress condition (example: design lot 2 at 60°C tested at week 3 is a specific "test condition"). A test condition was considered passing or failing according to acceptance criteria. Two sequential test conditions must have failed in order for the stability protocol to fail. Accelerated stability testing continued until the specified end point in the protocol was reached unless a specific test condition failed at two consecutive time points for any one parameter.

Three design lots of reagent were evaluated for each test condition. Each design lot was aliquoted into individual instrument reagent bottles and 12 bottles for each design lot were stored at each temperature condition. Temperature conditions were -20°C, ambient, 40°C, and 60°C. This allowed nine bottles total for testing (a bottle for Day 0 and eight additional bottles, one for each of the following weeks), leaving three extra bottles for any unforeseen circumstances. Each of the bottles was labeled with reagent information and storage temperature. Samples were removed from the bottles for analytical testing at each time point, and then the bottles were fitted with a stopper and tube for use in functional staining. If each critical parameter was determined to be within an acceptable range at 60°C at the 8-week time point, the Arrhenius model would predict 91-weeks of real-time stability.

Accelerated testing began for each design lot on Day 0 with testing of each critical parameter. After Day 0, the frequency of testing was dependent on the parameter being tested (see Table 4). Each time point for staining had 3 replicate slides. The slides stained on Day 0 were examined by a qualified reader to determine if the staining was acceptable. Analytical testing was performed at every time point.

A run was considered invalid (rather than failed) if the failure could be attributed to anything other than the reagents under test. Examples include but are not limited to: slide drying, cover slipping failures, tissue issues (degradation, sectioning), instrument or reagent vial malfunction, protocol programming error, or slide labeling/issues. Validity information was not be recorded for each slide.

### RESULTS AND DISCUSSION

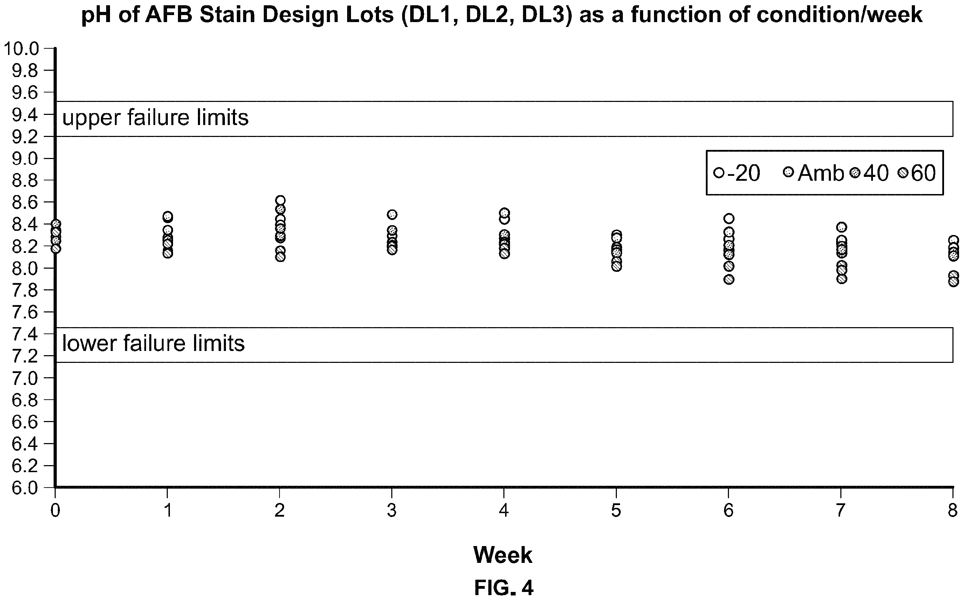

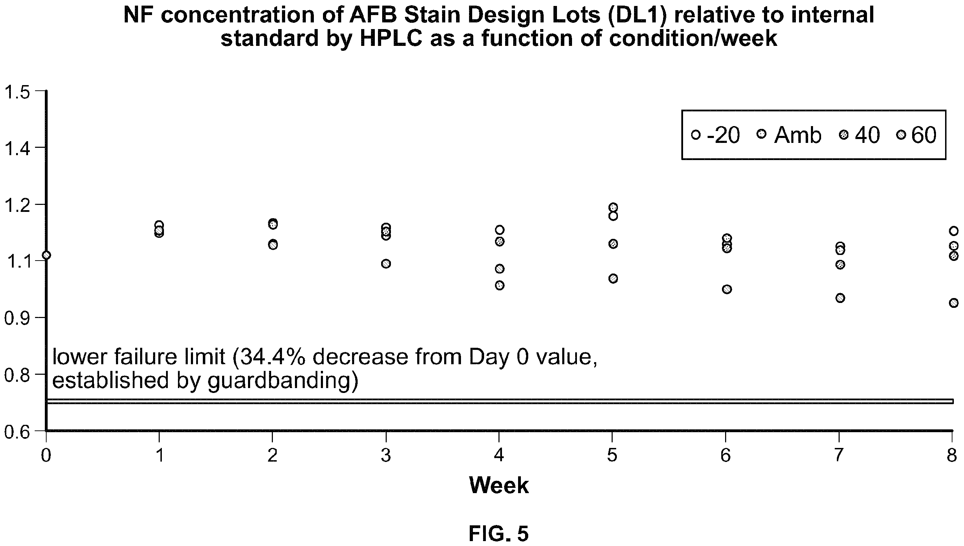

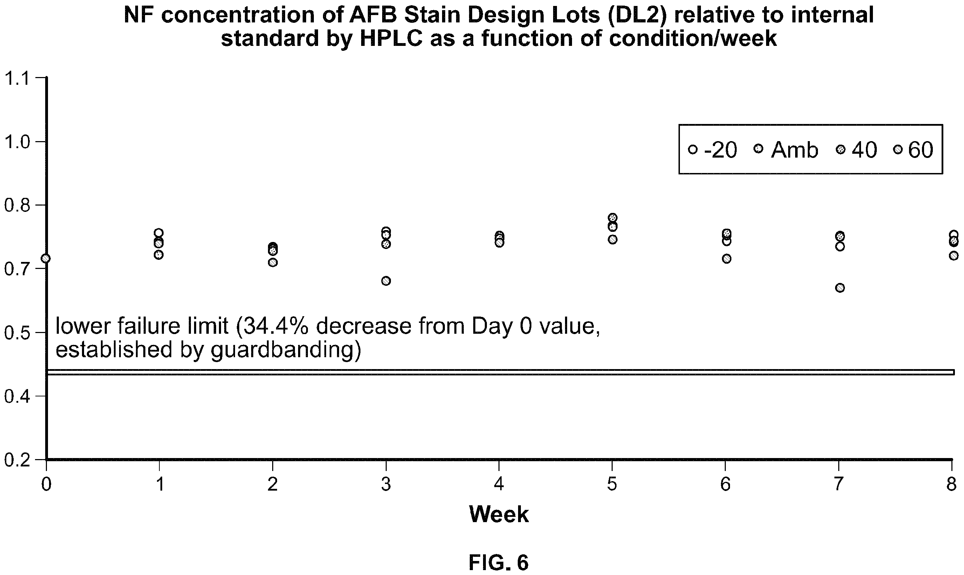

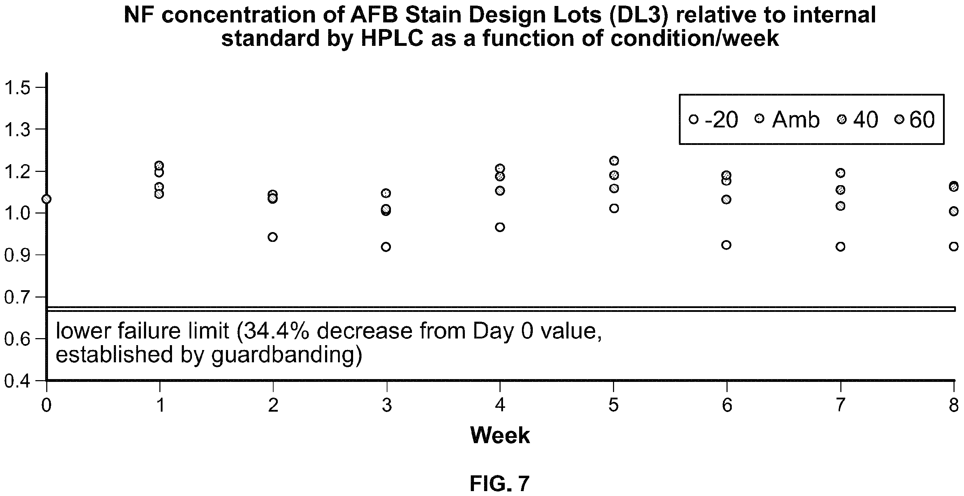

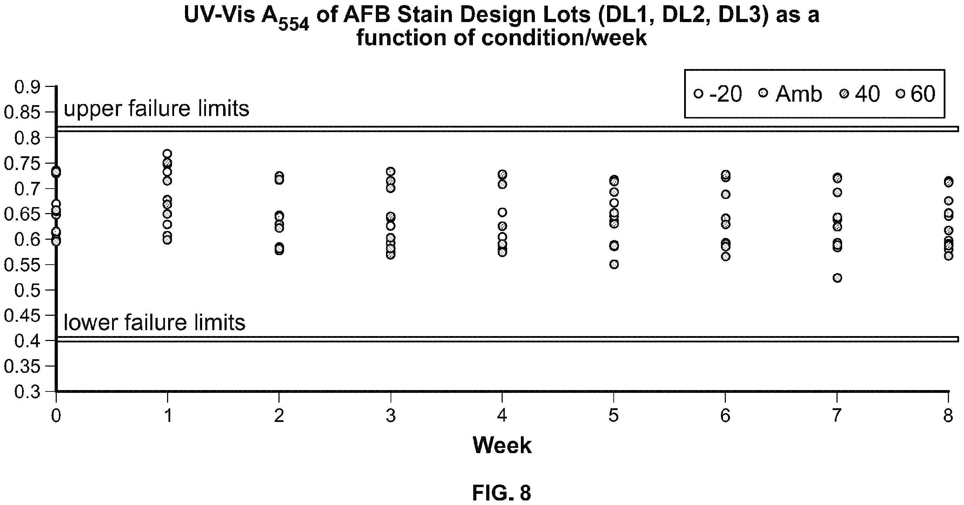

Accelerated stability testing was used to establish the initial product dating of the disclosed phenol-free AFB stain that contains weak base (e.g. TRIS base) and surfactant as lipophilic agents and is intended to supersede the on-market AFB stain, containing phenol and sourced from an external vendor. The performance of the design lot AFB stain solutions stored under each of the temperature conditions, ranging from -20°C to +60°C, was equivalent throughout the entire course of the 8-week accelerated stability study (equivalent to 91 weeks), as independently evaluated by the Pathology Office and a qualified reader for AFB. In addition to these results, analytical testing performed during this study (see Table 2 for each method) demonstrated that no parameter approached a pre-defined failure limit under any temperature condition. For pH, all measurements were within one pH unit from Day 0 (FIG. 4). Similarly, the HPLC and UV-Vis data demonstrated no significant changes in new fuchsin dye concentration (FIGS. 5 - 8). Finally, no indication of dye degradation (oxidation), which had been observed for a previous iteration of the AFB Stain formulation that contained KOH base instead of TRIS base, was observed under any condition evaluated in this study.

### CONCLUSIONS

No failure limits were approached in any of the analytical testing or functional staining performed during the course of this study for any of the four conditions tested (-20°C, ambient, +40°C, and +60°C storage of AFB Stain design lot solutions for 8 weeks). The data generated supports initial product dating of up to 91 weeks, using the Arrhenius model for condition 60°C. Overall, no indications of degradation were observed for the disclosed AFB solutions under any conditions, either ones that stress physical failure or chemical failure This study establishes initial product dating by demonstrating appropriate function of the disclosed AFB stain formulation.

**Table 2: Accelerated Stability Parameters Tested**

| | **Parameter** | **Method** | **Description** |
|---|---|---|---|
| **1** | Dye Concentration/ Degradation products | HPLC,MS, UV/vis | Measure possible change in the concentration of NF in solution. |
| | | | Detect known degradation products of NF |
| **2** | pH | pH | Track possible pH variations, indicative of chemical or physical changes |
| **3** | Functional Staining | BMKSS | Stain tissues to evaluate performance |

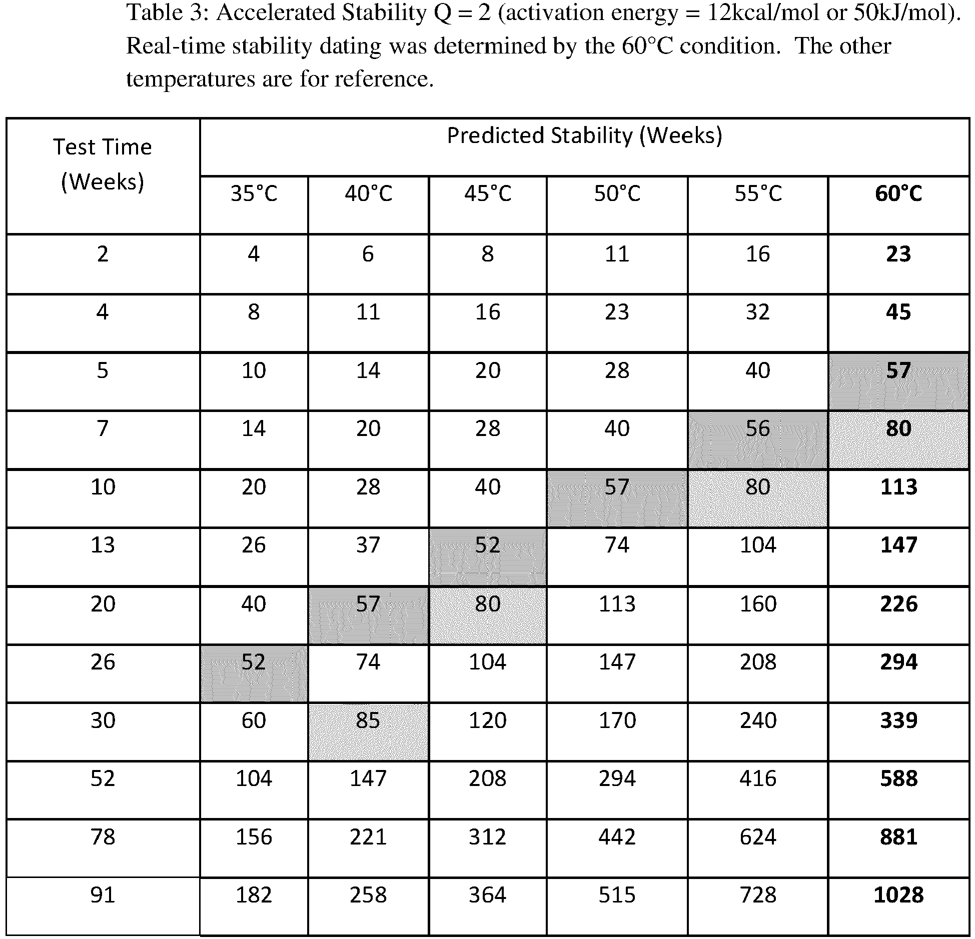

**Table 4: General testing schedule. Analytical testing occurs at each timepoint. Staining occurred at timepoint zero for all conditions, then as indicated in the Table. The schedule was applied to each of the three design lots.**

| | **Week** | | | | | | | | |
|---|---|---|---|---|---|---|---|---|---|
| **Storage Temp °C** | | **0 1 2** | | **3** | **4** | **5** | **6** | **7** | **8** |
| **-20** | XYZ | YZ | YZ | YZ | XYZ | XYZ | XYZ | XYZ | XYZ |
| **RT** | XYZ | YZ | YZ | YZ | XYZ | YZ | YZ | YZ | XYZ |
| **40** | XYZ | Y'Z | YZ | Y'Z | XYZ | Y'Z | YZ | YZ | XYZ |
| **60** | XYZ | Y'Z | YZ | Y'Z | XYZ | XYZ | XYZ | XYZ | XYZ |
| | | | | | | | | | |
| | | | X | STAINING | Y | HPLC, MS, UV/vis | Z | pH | |

### Additional Embodiments

In some embodiments, the staining system comprises components in addition to those described herein. Additional embodiments, features, systems, devices, materials, methods, and techniques which may be incorporated into the present systems and methods are described in U.S. Patent Nos. 8,911,815; 9,498,791; 9,618,430; 7,468,161; and 6,352,861. Yet additional components of staining systems are described in United States Patent Nos. 7,303,725, 8,048,373, 9,528,918, and 9,192,935.

In some embodiments, the staining system includes bulk fluid containers (e.g. to hold any of the solutions described herein prior to dispensing or applying to a sample). In some embodiments, slide processing apparatus, in some embodiments, further comprises a plurality of additional staining modules and a controller configured to independently control each of the staining modules.

In some embodiments, the staining system may include a frame supporting a stack of workstations comprising, for example, one or more drying or baking stations or modules, de-waxing or de-paraffinizing station or module, one or more staining stations or modules and a coverslipping station or module arranged in a tower. In some embodiments, a transport and elevator mechanism is provided adjacent to the tower for transporting a slide tray designed to carry a plurality of individual specimen bearing slides from a tray storage station through drying/baking, de-waxing, staining and coverslipping operations.

In some embodiments, a tray storage garage or station comprises a pair of stanchions bearing a plurality of vertically spaced shelves or skids for accommodating slide trays. In some embodiments, the storage station or garage includes a pivotally mounted door providing access to a first shelf position (for clarity, the outside skin or cover to garage has been omitted). A tray drive assembly indicated generally at including a pair of rotatably mounted drive wheels driven by a drive motor and transmission is positioned under the first shelf position for moving a tray into and out of the portal.

In some embodiments, the slide tray comprises a pan or slide tray having a generally rectangular plan, including a bottom wall, opposed side walls and opposed end walls. The slide tray typically is formed by conventional injection molding using synthetic polymers intended for such use, which are well-known in the art.

In some embodiments, the tray includes a specimen slide supporting rack for holding specimen slides in a substantially horizontal position in the same plane. Holding all the slides in the same plane facilitates baking and drying, as will be described below, and also prevents cross-contamination of slides during de-paraffinizing and staining as will be described below. In some embodiments, the rack includes a plurality of slide spring supports that limit the axial, lateral and vertical movement of specimen slides once placed on the slide tray. In some embodiments, the rack is supported above tray bottom at sufficient height to discourage or prevent the formation of films or bubbles forming between the specimen slide bottom and the tray bottom. In some embodiments, the slide spring supports hold the individual specimen slides in position by exerting force on opposing edges of the specimen slides. The floor of the slide tray is sloped towards the middle to facilitate drainage to a central location for evacuation of de-waxing fluids and stains, as will be described in detail hereinafter. In some embodiments, the tray permits the automated handling of a plurality of specimen slides through of the steps of drying/baking, de-paraffinizing, staining and coverslipping. In some embodiments, the tray includes splash rails and is arranged to accommodate 16 specimen slides arranged in a generally horizontal grid two slides wide and eight slides tall.

The staining module can include at least one heating element positioned to conductively heat the first sidewall, the second sidewall, or both. The slide holder can be used to heat the slide, specimen, and/or liquid while the band of liquid is manipulated across the specimen.

The controller, in some embodiments, includes one or more memories and a programmable processor. The memory stores a first sequence of program instructions and a second sequence of program instructions. The programmable processor is configured to execute the first sequence of program instructions in order to process a specimen on the slide with a first liquid and configured to execute the second sequence of program instructions to process the specimen with a second liquid that is different from the first liquid. In some embodiments, the programmable processor is configured to execute the first sequence of program instructions in order to heat the slide and the controller is configured to execute the second sequence of program instructions in order to heat the slide to a second temperature, the second temperature is different from the first temperature.

The controller, in some embodiments, is configured to execute a first sequence of program instructions to command the dispensing device to deliver a first liquid to the slide at a first rate. The controller is further configured to execute a second sequence of program instructions to command the dispensing device to deliver a second liquid to the slide at a second rate that is different from the first rate.

## Claims

1. An acid fast staining composition comprising a fuchsin, a weak base having a pKa ranging from between about 8 to about 20, a non-ionic surfactant, and an alcohol, wherein an amount of fuchsin in the composition ranges from between about 0.75 w/v% to about 2.75w/v% by total volume of the composition, wherein an amount of base in the composition ranges from between about 0.05 w/v% to about 0.5 w/v% by total volume of the composition, wherein an amount of surfactant in the composition ranges from between about 0.5 w/v% to about 4 w/v% by total volume of the composition, and wherein the acid fast staining composition is free from phenol.

2. The acid fast staining composition of claim 1, wherein the base is a hydroxide.

3. The acid fast staining composition of claim 1, wherein the base is tris(hydroxymethyl)aminomethane.

4. The acid fast staining composition of any of the preceding claims, wherein the non-ionic surfactant is an alcohol ethoxylate.

5. The acid fast staining composition of any of the preceding claims, wherein the non-ionic surfactant is a C₈-C₁₈ alcohol ethoxylate, optionally wherein the C8-C18 alcohol ethoxylate comprises less than 12 moles of ethylene oxide.

6. The acid fast staining composition of any of the preceding claims, wherein the fuchsin is new fuchsin.

7. The acid fast staining composition of any of the preceding claims, wherein a 10% aqueous solution of the acid fast staining composition has a pH ranging from between about 4 to about 6.

8. The acid fast staining composition of any of the preceding claims, wherein the base is selected from one which does not react with the fuchsin.

9. A biological sample stained with the acid fast staining composition of any of claims 1 to 8, optionally wherein the sample is free from phenol.

10. A container comprising the acid fast staining composition of any of claims 1 to 8.

11. A kit comprising: (a) a first composition comprising the acid fast staining composition of any of claims 1 to 8; and (b) a second composition selected from the group consisting of (i) a deparaffinization solution; (ii) a wash solution including a detergent; (iii) a decolorizing solution including a lower alcohol and an acid; and (iv) a secondary dye solution including a dye and a weak acid.

12. The kit of claim 11, wherein the first composition, the second composition and the secondary dye are each in separate containers.

13. An *in vitro* method of staining an acid fast organism in a biological sample, the method comprising the steps of:
(a) applying the acid fast staining composition of any of claims 1 to 8 to a biological sample obtained from a subject having or suspected of having an infection with an acid fast organism; and
(b) heating at least one of the acid fast staining composition or the biological sample to a temperature ranging from between about 30°C to about 45°C.

14. The method of claim 13, wherein the biological sample is incubated with the acid fast staining composition for a time period ranging from between about 10 minutes to about 40 minutes, optionally wherein the biological sample is incubated with the acid fast staining composition for a time period ranging from between about 12 minutes to about 24 minutes.

15. The method of claim 13 or 14, wherein between about 100 microliters to about 500 microliters of the acid fast staining composition is applied to the biological sample, optionally wherein about 200 microliters of the acid fast staining composition is applied to the biological sample.

16. The method of any of claims 13 to 15, further comprising the steps of
(c) applying a decolorizing solution including an alcohol and an acid to the biological sample; and
(d) applying a secondary stain including a dye and a weak acid to the biological sample.

17. The method of any of claims 13 to 16, wherein the acid fast staining composition is applied with an automated staining system.

## Patentansprüche

1. Säurefeste Färbezusammensetzung, umfassend ein Fuchsin, eine schwache Base mit einem pKa im Bereich von etwa 8 bis etwa 20, ein nichtionisches Tensid und einen Alkohol, wobei die Menge an Fuchsin in der Zusammensetzung im Bereich von etwa 0,75 Gew./Vol.-% bis etwa 2,75 Gew./Vol.-% bezogen auf das Gesamtvolumen der Zusammensetzung liegt, wobei die Menge an Base in der Zusammensetzung zwischen etwa 0,05 Gew./Vol.-% und etwa 0.5 Gew./Vol.-% bezogen auf das Gesamtvolumen der Zusammensetzung liegt, wobei die Menge an Tensid in der Zusammensetzung zwischen etwa 0,5 Gew./Vol.-% und etwa 4 Gew./Vol.-% bezogen auf das Gesamtvolumen der Zusammensetzung liegt, und wobei die säurefeste Färbezusammensetzung frei von Phenol ist.

2. Säurefeste Färbezusammensetzung nach Anspruch 1, wobei die Base ein Hydroxid ist.

3. Säurefeste Färbezusammensetzung nach Anspruch 1, wobei die Base Tris(hydroxymethyl)aminomethan ist.

4. Säurefeste Färbezusammensetzung nach einem der vorhergehenden Ansprüche, wobei das nichtionische Tensid ein Alkoholethoxylat ist.

5. Säurefeste Färbezusammensetzung nach einem der vorhergehenden Ansprüche, wobei das nicht-ionische Tensid ein C₈-C₁₈-Alkoholethoxylat ist, wobei das C₈-C₁₈-Alkoholethoxylat gegebenenfalls weniger als 12 Mol Ethylenoxid enthält.

6. Säurefeste Färbezusammensetzung nach einem der vorhergehenden Ansprüche, wobei das Fuchsin Neufuchsin ist.

7. Säurefeste Färbezusammensetzung nach einem der vorhergehenden Ansprüche, wobei eine 10%ige wässrige Lösung der säurefesten Färbezusammensetzung einen pH-Wert im Bereich von etwa 4 bis etwa 6 aufweist.

8. Säurefeste Färbezusammensetzung nach einem der vorangehenden Ansprüche, wobei die Base aus einer Base ausgewählt ist, die nicht mit dem Fuchsin reagiert.

9. Biologische Probe, die mit der säurefesten Färbezusammensetzung nach einem der Ansprüche 1 bis 8 gefärbt ist, wobei die Probe gegebenenfalls frei von Phenol ist.

10. Behälter, der die säurefeste Färbezusammensetzung nach einem der Ansprüche 1 bis 8 enthält.

11. Kit umfassend: (a) eine erste Zusammensetzung, umfassend die säurefeste Färbezusammensetzung nach einem der Ansprüche 1 bis 8; und (b) eine zweite Zusammensetzung, ausgewählt aus der Gruppe, bestehend aus (i) einer Entparaffinierungslösung; (ii) einer Waschlösung, die ein Detergens enthält; (iii) einer Entfärbungslösung, die einen niederen Alkohol und eine Säure enthält; und (iv) einer Sekundärfarbstofflösung, die einen Farbstoff und eine schwache Säure enthält.

12. Kit nach Anspruch 11, wobei sich die erste Zusammensetzung, die zweite Zusammensetzung und der Sekundärfarbstoff jeweils in separaten Behältern befinden.

13. In vitro-Verfahren zum Färben eines säurefesten Organismus in einer biologischen Probe, wobei das Verfahren die folgenden Schritte umfasst:
(a) Auftragen der säurefesten Färbezusammensetzung nach einem der Ansprüche 1 bis 8 auf eine biologische Probe, die von einem Individuum erhalten wurde, das eine Infektion mit einem säurefesten Organismus hat oder dessen Infektion damit vermutet wird; und
(b) Erhitzen der säurefesten Färbezusammensetzung und/oder der biologischen Probe auf eine Temperatur im Bereich zwischen etwa 30°C und etwa 45°C.

14. Verfahren nach Anspruch 13, wobei die biologische Probe mit der säurefesten Färbezusammensetzung für eine Zeitspanne zwischen etwa 10 Minuten und etwa 40 Minuten inkubiert wird, wobei die biologische Probe gegebenenfalls mit der säurefesten Färbezusammensetzung für eine Zeitspanne zwischen etwa 12 Minuten und etwa 24 Minuten inkubiert wird.

15. Verfahren nach Anspruch 13 oder 14, wobei zwischen etwa 100 Mikroliter bis etwa 500 Mikroliter der säurefesten Färbezusammensetzung auf die biologische Probe aufgebracht werden, wobei gegebenenfalls etwa 200 Mikroliter der säurefesten Färbezusammensetzung auf die biologische Probe aufgebracht werden.

16. Verfahren nach einem der Ansprüche 13 bis 15, das ferner die folgenden Schritte umfasst:
(c) Auftragen einer Entfärbungslösung, die einen Alkohol und eine Säure enthält, auf die biologische Probe; und
(d) Aufbringen einer Sekundärfärbung, die einen Farbstoff und eine schwache Säure enthält, auf die biologische Probe.

17. Verfahren nach einem der Ansprüche 13 bis 16, wobei die säurefeste Färbezusammensetzung mit einem automatischen Färbesystem aufgebracht wird.

## Revendications

1. Composition de coloration acido-résistante comprenant une fuchsine, une base faible possédant un pKa compris entre environ 8 et environ 20, un tensioactif non ionique, et un alcool, dans laquelle une quantité de fuchsine dans la composition est comprise entre environ 0,75 % p/v et environ 2,75 % p/v en volume total de la composition, dans laquelle une quantité de base dans la composition est comprise entre environ 0,05 % p/v et environ 0,5 % p/v en volume total de la composition, dans laquelle une quantité de tensioactif dans la composition est comprise entre environ 0,5 % p/v et environ 4 % p/v en volume total de la composition, et dans laquelle la composition de coloration acido-résistante est exempte de phénol.

2. Composition de coloration acido-résistante selon la revendication 1, dans laquelle la base est un hydroxyde.

3. Composition de coloration acido-résistante selon la revendication 1, dans laquelle la base est le tris(hydroxyméthyl)aminométhane.

4. Composition de coloration acido-résistante selon l'une quelconque des revendications précédentes, dans laquelle le tensioactif non ionique est un éthoxylate d'alcool.

5. Composition de coloration acido-résistante selon l'une quelconque des revendications précédentes, dans laquelle le tensioactif non ionique est un éthoxylate d'alcool en C₈-C₁₈, facultativement dans laquelle l'éthoxylate d'alcool en C₈-C₁₈ comprend moins de 12 moles d'oxyde d'éthylène.

6. Composition de coloration acido-résistante selon l'une quelconque des revendications précédentes, dans laquelle la fuchsine est une nouvelle fuchsine.

7. Composition de coloration acido-résistante selon l'une quelconque des revendications précédentes, dans laquelle une solution aqueuse à 10 % de la composition de coloration acido-résistante possède un pH compris entre environ 4 et environ 6.

8. Composition de coloration acido-résistante selon l'une quelconque des revendications précédentes, dans laquelle la base est sélectionnée à partir d'une base qui ne réagit pas avec la fuchsine.

9. Échantillon biologique coloré avec la composition de coloration acido-résistante selon l'une quelconque des revendications 1 à 8, facultativement dans lequel l'échantillon est exempt de phénol.

10. Récipient comprenant la composition de coloration acido-résistante selon l'une quelconque des revendications 1 à 8.

11. Kit comprenant : (a) une première composition comprenant la composition de coloration acido-résistante selon l'une quelconque des revendications 1 à 8 ; et (b) une seconde composition sélectionnée dans le groupe consistant en (i) une solution de déparaffinage ; (ii) une solution de lavage comprenant un détergent ; (iii) une solution de décoloration comprenant un alcool inférieur et un acide ; et (iv) une solution de colorant secondaire comprenant un colorant et un acide faible.

12. Kit selon la revendication 11, dans lequel la première composition, la seconde composition et le colorant secondaire sont chacun dans des récipients distincts.

13. Procédé *in vitro* de coloration d'un organisme acido-résistant dans un échantillon biologique, le procédé comprenant les étapes consistant à :
(a) appliquer la composition de coloration acido-résistante selon l'une quelconque des revendications 1 à 8 à un échantillon biologique obtenu chez un sujet présentant ou suspecté de présenter une infection par un organisme acido-résistant ; et
(b) chauffer au moins l'un de la composition de coloration acido-résistante ou de l'échantillon biologique à une température comprise entre environ 30 °C et environ 45 °C.

14. Procédé selon la revendication 13, dans lequel l'échantillon biologique est incubé avec la composition de coloration acido-résistante pendant une période comprise entre environ 10 minutes et environ 40 minutes, facultativement dans lequel l'échantillon biologique est incubé avec la composition de coloration acido-résistante pendant une période comprise entre environ 12 minutes et environ 24 minutes.

15. Procédé selon la revendication 13 ou 14, dans lequel entre environ 100 microlitres et environ 500 microlitres de la composition de coloration acido-résistante sont appliqués à l'échantillon biologique, facultativement dans lequel environ 200 microlitres de la composition de coloration acido-résistante sont appliqués à l'échantillon biologique.

16. Procédé selon l'une quelconque des revendications 13 à 15, comprenant en outre les étapes consistant à
(c) appliquer une solution de décoloration comprenant un alcool et un acide à l'échantillon biologique ; et
(d) appliquer un colorant secondaire comprenant un colorant et un acide faible à l'échantillon biologique.

17. Procédé selon l'une quelconque des revendications 13 à 16, dans lequel la composition de coloration acido-résistante est appliquée à l'aide d'un système de coloration automatisé.
